# EUROPÄISCHE PATENTANMELDUNG

(11) **EP 1 769 778 A1**
(43) Veröffentlichungstag der Anmeldung: **04.04.2007**
(21) Anmeldenummer: 06020071.4
(22) Anmeldetag: 26.09.2006
(51) Int. Cl.: A61F 2/90

(54) **MR-kompatible Gefäßendoprothese**

(30) Priorität: 01.10.2005 DE 102005047235
(71) Anmelder: Grönemeyer, Dietrich H.W., 45549 Sprockhövel (DE)
(72) Erfinder: Busch, Martin, Dr., 58455 Witten (DE); Grönemeyer, Dietrich H.W., Prof. Dr. med., 45549 Sprockhövel (DE)
(74) Vertreter: Isfort, Olaf

(57) **Zusammenfassung**

Die Erfindung betrifft ein medizinisches Implantat oder Instrument, insbesondere eine Gefäßendoprothese (1), mit einem radial aufweitbaren Schlauchteil (2), das aus einem schlauchförmigen Gestricke mit ineinander greifenden Maschen aus einer oder mehreren Einzelfasern (9) besteht. Zur Bereitstellung eines solchen Implantates oder Instrumentes, das eine MR-Bildgebung des Inneren des Implantates oder Instrumentes ermöglicht, schlägt die Erfindung vor, dass die Fasern (9) des Gestrickes elektrisch leitend sind und eine Induktivität (7, 8) innerhalb eines Hochfrequenzschwingkreises ausbilden.

## Beschreibung

Die Erfindung betrifft ein medizinisches Implantat oder Instrument, insbesondere eine Gefäßendoprothese, mit einem radial aufweitbaren Schlauchteil, das aus einem schlauchförmigen Gestricke mit ineinander greifenden Maschen aus einer oder mehreren Einzelfasern besteht.

Gefäßendoprothesen, so genannte Stents und andere medizinische Implantate oder Instrumente, die ein radial aufweitbares Schlauchteil aufweisen, wie z. B. intravaskuläre Filtersysteme oder Gefäßklappen, sind aus dem Stand der Technik bekannt. Das radial aufweitbare Schlauchteil wird bei Stents üblicherweise durch gitterförmig angeordnete Metallfilamente gebildet, die zur Abstützung und/oder Glättung einer geschädigten Koronargefäßwand eingesetzt werden. Ein Stent wird - ähnlich wie bei einer PTCA-Behandlung - mittels eines Ballonkatheters im Bereich der geschädigten Stelle des zu behandelnden Gefäßes radial aufgeweitet und dadurch fixiert. Stents werden hauptsächlich eingesetzt, um akute Gefäßverschlüsse oder Restenosierungen nach PTCA-Behandlungen zu verhindern. So genannte Stent-Grafts werden zur Behandlung von Aneurysmen eingesetzt.

Aus der EP 0 292 587 B1 ist eine Gefäßendoprothese mit einem radial aufweitbaren, flexiblen Schlauchteil vorbekannt, wobei das Schlauchteil aus einem schlauchförmigen Gestricke mit ineinander greifenden Maschen aus einer oder mehreren Einzelfasern besteht. Vorteilhaft ist bei der vorbekannten Gefäßendoprothese, dass diese im nicht aufgeweiteten und auch im aufgeweiteten Zustand sehr flexibel ist. Das schlauchförmige Gestricke hat den Vorteil, dass das daraus bestehende Schlauchteil innerhalb bestimmter Grenzen stufenlos radial aufweitbar und somit problemlos an die individuellen Gegebenheiten des jeweils zu behandelnden Patienten anpassbar ist. Vor deren bestimmungsgemäßer Verwendung kann die Endoprothese im Durchmesser stark reduziert sein, da die einzelnen Maschen des Gestrickes mit Spiel ineinander greifen. Beim Aufweiten des Gestrickes erfahren die Faserabschnitte innerhalb der ineinander greifenden Maschen des Gestrickes eine plastische Verformung, die bewirkt, dass die Endoprothese in ihrer Aufweitlage verharrt, ohne dass weitere Maßnahmen zur Fixierung notwendig sind. Gefäßendoprothesen mit einem radial aufweitbaren Schlauchteil, das aus einem Gestricke besteht, haben somit die Vorteile der hohen Flexibilität bei gleichzeitig gutem Beharrungsvermögen in der Aufweitlage.

Die diagnostische Bildgebung von Bereichen in der Umgebung einer Gefäßendoprothese oder eines ähnlichen medizinischen Implantates mittels magnetischer Resonanz (MR) erweist sich oft als problematisch. Dies kann zum einen darauf zurückzuführen sein, dass das im Körper des untersuchten Patienten befindliche Implantat aus paramagnetischem Material besteht. Durch die magnetische Suszeptibilität des Implantats wird das Magnetfeld in der ansonsten diamagnetischen Umgebung des Implantats verzerrt, sodass in den aufgenommenen Bildern Artefakte entstehen. Diese artefaktbehafteten Bilder sind dann für diagnostische Zwecke zumeist nicht brauchbar. Medizinische Implantate und Instrumente, die, wie dies bei Stents häufig der Fall ist, ein aus einer metallischen Gitterstruktur bestehendes Schlauchteil aufweisen, haben zudem den Nachteil, dass die gitter- oder netzartige Struktur bei der MR-Bildgebung als Faraday-Käfig wirkt, sodass die bei der MR-Bildgebung eingestrahlten Hochfrequenzfelder nicht in das Volumen innerhalb des Implantats eindringen. Aufgrund dieser Abschirmung bleibt das Innere eines herkömmlichen Stents bei der MR-Bildgebung nachteiligerweise unsichtbar. Nachteilig ist dies insbesondere, weil verhindert wird, dass mittels MR-Bildgebung eine Restenosierung im Inneren eines Stents frühzeitig diagnostiziert werden kann.

Davon ausgehend liegt der Erfindung die Aufgabe zugrunde, ein medizinisches Implantat oder Instrument, insbesondere eine Gefäßendoprothese, derart weiterzuentwickeln, dass eine MR-Bildgebung, insbesondere des Inneren des Implantats möglich ist.

Diese Aufgabe löst die Erfindung ausgehend von einem medizinischen Implantat oder Instrument der eingangs genannten Art dadurch, dass die Fasern des Gestrickes elektrisch leitend sind und eine Induktivität innerhalb eines Hochfrequenzschwingkreises ausbilden.

Gemäß der Erfindung wird also das radial aufweitbare Schlauchteil aus elektrisch leitenden Fasern hergestellt, die eine Induktivität innerhalb eines Hochfrequenzschwingkreises ausbilden. Dadurch wird die gewünschte MR-Kompatibilität erzielt. Das Gestricke des aufweitbaren Schlauchteils hat gemäß der Erfindung eine elektrische Impedanz. So entsteht insgesamt eine Resonatorstruktur, die bei der MR-Bildgebung genutzt werden kann. Hierzu muss die Resonanzfrequenz des Hochfrequenzschwingkreises auf die Resonanzfrequenz des benutzen MR-Gerätes abgestimmt sein. Die bei der MR-Bildgebung eingestrahlten Hochfrequenzfelder werden dann nicht, wie bei herkömmlichen Stents, abgeschirmt, sondern - im Gegensteil - im Inneren des Implantats sogar verstärkt. Damit ist dann die MR-Bildgebung des Volumens innerhalb des Schlauchteils des erfindungsgemäßen Implantats besonders gut möglich.

Zweckmäßigerweise sind bei dem erfindungsgemäßen medizinischen Implantat oder Instrument die Fasern des Gestrickes in den Kreuzungspunkten der Maschen gegeneinander elektrisch isoliert. Mit Vorteil können die Fasern Metalldrähte sein, die eine elektrisch isolierende Beschichtung aufweisen. Die Isolierung in den Kreuzungspunkten ist erforderlich, damit die Induktivität der Resonatorstruktur in der erforderlichen Weise eingestellt werden kann. Ohne Isolierung käme es zu Kurzschlüssen im Bereich der Kreuzungspunkte. Gemäß einer möglichen Ausführungsform der Erfindung können die Fasern in zumindest einigen der Kreuzungspunkte der Maschen elektrisch leitend miteinander verbunden sein. An einigen der Kreuzungspunkte können gezielt elektrische Verbindungen geschaffen werden, um die Induktivität auf den gewünschten Wert einzustellen, damit die Resonatorstruktur auf die Resonanzfrequenz des benutzen MR-Gerätes abgestimmt ist. Die gezielte elektrisch leitende Verbindung in zumindest einigen der Kreuzungspunkte kann genutzt werden, um Strompfade innerhalb des Gestrickes des Schlauchteils zu definieren. Der Verlauf dieser Strompfade bestimmt dann die Impedanz.

Gemäß einer sinnvollen Ausgestaltung des erfindungsgemäßen medizinischen Implantates oder Instrumentes ist das Gestricke derart hergestellt, dass die Fasern keine in sich geschlossenen Strompfade innerhalb des Gestrickes ausbilden. Dadurch wird verhindert, dass sich auf der Oberfläche des Schlauchteils bei der MR-Bildgebung Wirbelströme aufgrund der von außen eingestrahlten Hochfrequenzfelder ausbilden. Somit schirmt das erfindungsgemäße medizinische Implantat die eingestrahlten Hochfrequenzfelder nicht ab. Das Schlauchteil kann nicht als Faraday-Käfig wirken.

Gemäß einer vorteilhaften Ausgestaltung des erfindungsgemäßen medizinischen Implantates oder Instrumentes kann das Gestricke des Schlauchteils derart hergestellt sein, dass die Fasern entlang der Längserstreckung des Schlauchteils wenigstens einen helixförmigen Strompfad oder Strompfadabschnitt ausbilden. Es ist beispielsweise möglich, das gesamte Schlauchteil durch Stricken aus einem durchgehenden, elektrisch isolierten Metalldraht herzustellen. Im Ergebnis wird so eine helixförmige Drahtstruktur ausgebildet, die die Eigenschaften einer Solenoid-Spule hat und damit besonders gut zur Erzeugung einer Induktivität geeignet ist. Voraussetzung ist, dass die Faserabschnitte in den Kreuzungspunkten der Maschen gegeneinander elektrisch isoliert sind. Wie oben beschrieben, können an einzelnen Kreuzungspunkten der Maschen gezielt elektrisch leitende Verbindungen hergestellt werden, um beispielsweise die Zahl der effektiven Windungen der Solenoid-Spule vorzugeben und dadurch die Induktivität einzustellen. Die Erzeugung einer Sattelspule durch gezielte elektrisch leitende Verbindung der Faserabschnitte ist ebenfalls denkbar.

Wenn gemäß der Erfindung das Schlauchteil des medizinischen Implantates oder Instrumentes nach Art einer Solenoid- oder Sattelspule ausgebildet ist, ist die Polarisationsebene des bei der MR-Bildgebung im Inneren des Schlauchteils erzeugten Hochfrequenzfeldes parallel zur Längserstreckung des Schlauchteils.

Bei einer Gefäßendoprothese kann es, je nach Lage der Prothese im Körper des Patienten, zur Bildgebung des Inneren der Prothese erforderlich sein, dass das Hochfrequenzfeld in einer Ebene senkrecht zur Längserstreckung des Schlauchteils polarisiert ist. In diesem Fall ist das Gestricke des Schlauchteils sinnvollerweise derart hergestellt, dass die Fasern wenigstens einen parallel zur Längserstreckung des Schlauchteils verlaufenden Strompfad oder Strompfadabschnitt ausbilden. Dabei können besonders vorteilhaft zwei oder mehr parallel zur Längserstreckung des Schlauchteils verlaufende Strompfade oder Strompfadabschnitte über den Umfang des Schlauchteils verteilt angeordnet sein. Bei dieser Ausgestaltung wird eine Resonatorstruktur nach Art eines Käfigresonators (engl. "birdcage coil") erzeugt. Bei einer solchen Resonatorstruktur sind die parallel zur Längserstreckung des Resonators verlaufenden Strompfande an den Stirnseiten des Resonators über Kapazitäten miteinander verbunden. Ebenso können Kapazitäten in die über den Umfang des Resonators verteilten Strompfade zwischengeschaltet sein.

Gemäß einer besonders vorteilhaften Ausführungsform besteht das Schlauchteil des erfindungsgemäßen medizinischen Implantates oder Instrumentes aus wenigstens zwei einander umschließenden Gestrickelagen. Die einander umschließenden Gestrickelagen können durchaus unterschiedlich ausgeführt sein. Demgemäß besteht eine besonders hohe Flexibilität hinsichtlich der Einstellung der Induktivität der Resonatorstruktur. Außerdem hat die mehrlagige Ausgestaltung des Schlauchteils vorteilhaft eine erhöhte Widerstandsfähigkeit gegen äußere Krafteinwirkung.

Das Schlauchteil des erfindungsgemäßen medizinischen Implantates oder Instrumentes ist durch Stricken, Häkeln, Weben, Knüpfen oder andere maschenbildende Verfahren aus den Einzelfasern herstellbar. Die verschiedensten bekannten Strick-, Häkel-, Web- und Knüpfmuster kommen in Frage. Das Muster wird je nach den gewünschten Eigenschaften der zu erzeugenden Resonatorstruktur ausgewählt.

Vorteilhaft ist es weiterhin, wenn das Gestricke des Schlauchteils bei dem erfindungsgemäßen medizinischen Implantat oder Instrument ein loses Ende aufweist, sodass das Gestricke durch Zug an dem losen Ende auflösbar ist. Das Auflösen eines Gestrickes durch Zug an einem losen Ende wird auch als "Aufribbeln" bezeichnet. Heutzutage geht man vielfach dazu über, Gefäßendoprothesen nach einer bestimmten Zeit aus dem behandelten Blutgefäß wieder zu entfernen. Bei dem erfindungsgemäßen Implantat kann dies in besonders einfacher Weise durch Aufribbeln des Gestrickes erfolgen.

Ausführungsbeispiele der Erfindung werden im Folgenden unter Bezugnahme auf die Zeichnungen näher erläutert. Es zeigen:
- Fig. 1: dreidimensionale schematische Ansicht einer erfindungsgemäßen Gefäßendoprothese:
- Fig. 2: Ersatzschaltbild zur Verdeutlichung der Resonatoreigenschaften des erfindungsgemäßen Implantats;
- Fig. 3: vergrößerte Ausschnittdarstellung des Gestrickes des erfindungsgemäßen Implantats gemäß einer ersten Variante;
- Fig. 4: Ausschnittdarstellung des Gestrickes gemäß einer zweiten Variante;
- Fig. 5: schematische Darstellung einer erfindungsgemäßen Resonatorstruktur.

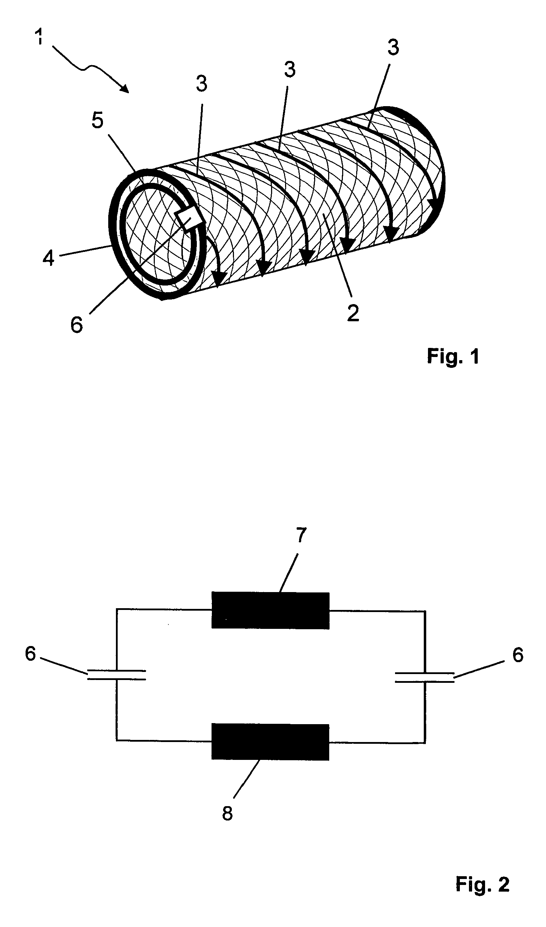

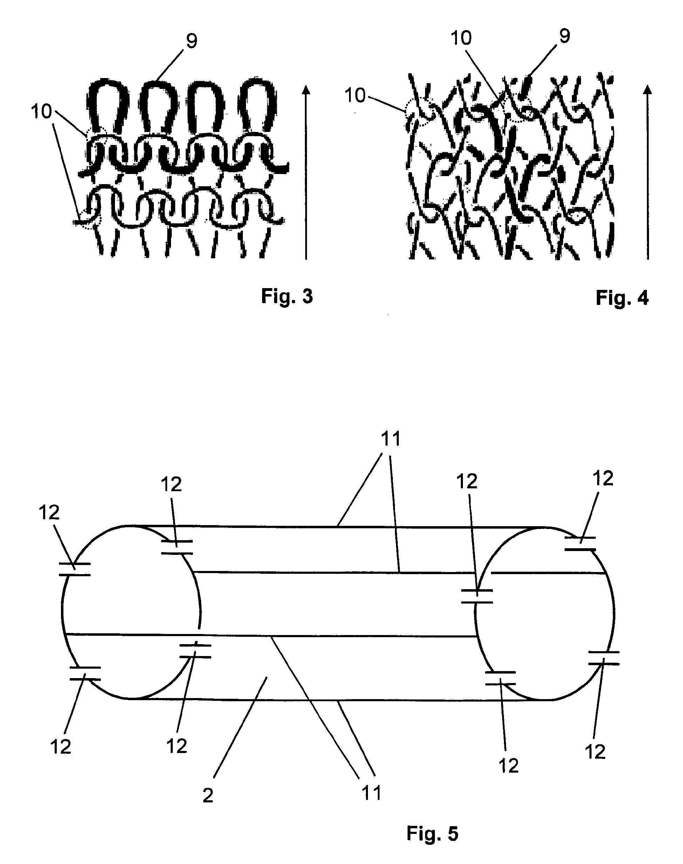

Bei dem in der Fig. 1 dargestellten Implantat handelt es sich um einen Stent, der als Ganzes mit der Bezugsziffer 1 bezeichnet ist. Der Stent 1 weist ein radial aufweitbares Schlauchteil 2 auf, das aus einem schlauchförmigen Gestricke mit ineinander greifenden Maschen aus einer oder mehreren Einzelfasern besteht. Die Struktur des Gestrickes wird weiter unten unter Bezugnahme auf die Fig. 3 und 4 näher erläutert. Die Fasern des Gestrickes sind elektrisch leitend und bilden eine Induktivität innerhalb eines Hochfrequenzschwingkreises aus. Das Gestricke ist derart hergestellt, dass die Fasern entlang der Längserstreckung des Schlauteils 2 einen helixförmigen Strompfad 3 ausbilden, der sich durchgehend von einem Ende des Schlauchteils 2 zum anderen Ende hin erstreckt. Bei dem in der Fig. 1 dargestellten Ausführungsbeispiel besteht das Schlauchteil 2 aus zwei Gestrickelagen, nämlich aus einer äußeren Gestrickelage 4 und einer inneren Gestrickelage 5. Das Gestricke der inneren Gestrickelage 5 ist ebenfalls so ausgeführt, dass ein helixförmiger Strompfad ausgebildet ist, der sich von einem Ende des Schlauchteils 2 zum gegenüberliegenden Ende hin erstreckt. Dabei ist der Drehsinn der Helix der inneren Gestrickelage 5 entgegengesetzt zu dem Drehsinn der Helix der äußeren Gestrickelage 4. Die von den Fasern der beiden Gestrickelagen 4 und 5 gebildeten Strompfade sind über Kapazitäten 6 an den Enden des Schlauchteils 2 miteinander verbunden. In der Fig. 1 ist nur die Kapazität am vorderen Ende des Schlauchteils 2 zu erkennen. Die Kapazitäten 6 müssen nicht zwingend als separate Schaltelemente ausgeführt sein. Die Kapazitäten 6 können auch durch die übereinanderliegenden, elektrisch leitenden Bereiche der Gestrickelagen 4 und 5 gebildet sein.

Anhand des Schaltbildes gemäß Fig. 2 wird deutlich, wie die von den Gestrickelagen 4 und 5 ausgebildeten Induktivitäten 7 und 8 über die Kapazitäten 6 miteinander verschaltet sind. Die Induktivität 7 ist der äußeren Gestrickelage 4 des Stents 1 zugeordnet, während die Induktivität 8 der inneren Gestrickelage 5 zugeordnet ist. Insgesamt entsteht so ein Resonator, nämlich ein Hochfrequenzschwingkreis, wobei die Induktivitäten 7 und 8 und die Kapazitäten 6 so aufeinander abgestimmt sind, dass die Resonanzfrequenz derjenigen eines MR-Gerätes angepasst ist.

Die Fig. 3 und 4 sind ebene Darstellungen eines Ausschnitts aus dem Gestricke des radial aufweitbaren Schlauchteils 2 der erfindungsgemäßen Gefäßendoprothese 1. Die Fig. 3 zeigt ein Muster mit ineinander greifenden Maschen, das aus einem einzelnen elektrisch leitenden Draht 9, beispielsweise durch Umstricken eines zylindrischen Kerns, herstellbar ist. In Kreuzungspunkten 10 der Maschen sind die Abschnitte des Drahtes 9 gegeneinander elektrisch isoliert. Mit dem in der Fig. 3 dargestellten Muster kann ein zylindrisches Gestricke gebildet werden, bei dem entlang der Längserstreckung des Schlauchteils 2 im Ergebnis ein durchgehender helixförmiger Strompfad gebildet werden, der sich von einem Ende des Schlauchteils 2 zum gegenüberliegenden Ende hin erstreckt. Die Fig. 4 zeigt ein weiteres mögliches Gestrickemuster, bei dem die einzelnen Maschen kettenartig ineinander greifen. Der Verlauf der Einzelfasern innerhalb des Gestrickes kann der Fig. 4 gut anhand der fett dargestellten Einzelfaser 9 entnommen werden. Das in der Fig. 4 dargestellte Muster eignet sich gut zur Herstellung eines Gestrickes, bei dem die Fasern 9 des Gestrickes mehrere parallel zur Längserstreckung des Schlauchteils 2 verlaufende Strompfade oder Strompfadabschnitte ausbilden. In den Fig. 3 und 4 ist die Richtung der Längserstreckung des Schlauchteils 2 durch die Pfeile angedeutet. Auch bei dem in der Fig. 4 dargestellten Muster sind die Fasern 9 in den Kreuzungspunkten 10 der Maschen gegeneinander elektrisch isoliert. Die in den Fig. 3 und 4 dargestellten Muster können besonders einfach aus Metalldrähten 9 hergestellt werden, die eine elektrisch isolierende Beschichtung aufweisen.

In der Fig. 5 ist schematisch eine Resonatorstruktur dargestellt, die gemäß der Erfindung beispielsweise mittels eines Schlauchteils 2 aus einem Gestricke, wie es in der Fig. 4 dargestellt ist, herstellbar ist. Dabei sind mehrere Strompfade gleichmäßig über den Umfang des Schlauchteils 2 verteilt angeordnet. An den gegenüberliegenden Enden des Schlauchteils 2 sind die von den Fasern 9 des Gestrickes gebildeten Strompfade 11 über Kapazitäten 12 miteinander verbunden. Im Ergebnis entsteht ein Käfigresonator, dessen Resonanzfrequenz über entsprechende Abstimmung der Kapazitäten 12 auf die Resonanzfrequenz des benutzten MR-Gerätes abgestimmt ist. Der Käfigresonator hat den Vorteil, dass die Polarisationsebene des innerhalb des Resonators erzeugten Hochfrequenzfeldes senkrecht zur Längsersteckung des Schlauchteils 2 verläuft. Eine derartige Resonatorstruktur ermöglicht es, das Innere des Stents 1 mittels MR-Bildgebung zu untersuchen, wenn sich der Stent in einem im Wesentlichen parallel zur Körperlängsachse des Patienten verlaufenden Blutgefäß befindet. Bei den häufig benutzen geschlossen MR-Geräten verläuft nämlich das externe statische Magnetfeld ebenfalls parallel zur Körperlängsachse. Für die Bildgebung ist es wichtig, dass das Hochfrequenzfeld in einer Ebene senkrecht zur Richtung des statischen Magnetfeldes polarisiert ist.

## Patentansprüche

1. Medizinisches Implantat oder Instrument, insbesondere Gefäßendoprothese (1), mit einem radial aufweitbaren Schlauchteil (2), das aus einem schlauchförmigen Gestricke mit ineinander greifenden Maschen aus einer oder mehreren Einzelfasern (9) besteht, **dadurch gekennzeichnet, dass** die Fasern (9) des Gestrickes elektrisch leitend sind und eine Induktivität (7, 8) innerhalb eines Hochfrequenzschwingkreises ausbilden.

2. Medizinisches Implantat oder Instrument nach Anspruch 1, **dadurch gekennzeichnet, dass** die Fasern (9) in den Kreuzungspunkten (10) der Maschen gegeneinander elektrisch isoliert sind.

3. Medizinisches Implantat oder Instrument nach Anspruch 1 oder 2, **dadurch gekennzeichnet, dass** die Fasern (9) Metalldrähte sind, die eine elektrisch isolierende Beschichtung aufweisen.

4. Medizinisches Implantat oder Instrument nach einem der Ansprüche 1 bis 3, **dadurch gekennzeichnet, dass** die Fasern (9) in zumindest einigen der Kreuzungspunkte (10) der Maschen elektrisch leitend miteinander verbunden sind.

5. Medizinisches Implantat oder Instrument nach einem der Ansprüche 1 bis 4, **dadurch gekennzeichnet, dass** das Gestricke derart hergestellt ist, dass die Fasern (9) keine in sich geschlossenen Strompfade innerhalb des Gestrickes ausbilden.

6. Medizinisches Implantat oder Instrument nach einem der Ansprüche 1 bis 5, **dadurch gekennzeichnet, dass** das Gestricke derart hergestellt ist, dass die Fasern (9) entlang der Längserstreckung des Schlauchteils (2) wenigstens einen helixförmigen Strompfad (3) oder Strompfadabschnitt ausbilden.

7. Medizinisches Implantat oder Instrument nach einem der Ansprüche 1 bis 5, **dadurch gekennzeichnet, dass** das Gestricke derart hergestellt ist, dass die Fasern (9) wenigstens einen parallel zur Längserstreckung des Schlauchteils (2) verlaufenden Strompfad (11) oder Strompfadabschnitt ausbilden.

8. Medizinisches Implantat oder Instrument nach Anspruch 7, **dadurch gekennzeichnet, dass** zwei oder mehr parallel zur Längserstreckung des Schlauchteils (2) verlaufende Strompfade (11) oder Strompfadabschnitte über den Umfang des Schlauchteils (2) verteilt angeordnet sind.

9. Medizinisches Implantat oder Instrument nach einem der Ansprüche 1 bis 8, **dadurch gekennzeichnet, dass** die von den Fasern (9) gebildeten Strompfade (3, 11) oder Strompfadabschnitte über wenigstens eine elektrische Kapazität (6, 12) miteinander verbunden sind.

10. Medizinisches Implantat oder Instrument nach einem der Ansprüche 1 bis 9, **dadurch gekennzeichnet, dass** das Schlauchteil (2) aus wenigstens zwei einander umschließenden Gestrickelagen (4, 5) besteht.

11. Medizinisches Implantat oder Instrument nach einem der Ansprüche 1 bis 10, **dadurch gekennzeichnet, dass** das Schlauchteil (2) durch Stricken, Häkeln, Weben, Knüpfen oder andere maschenbildende Verfahren aus den Einzelfasern (9) herstellbar ist.

12. Medizinisches Implantat oder Instrument nach einem der Ansprüche 1 bis 11, **dadurch gekennzeichnet, dass** das Gestricke des Schlauchteils (2) ein loses Ende aufweist, sodass das Gestricke durch Zug an dem losen Ende auflösbar ist.
